# EUROPÄISCHE PATENTSCHRIFT

(11) **EP 3 134 075 B1**
(45) Veröffentlichungstag und Bekanntmachung des Hinweises auf die Patenterteilung: **16.08.2017**
(21) Anmeldenummer: 15717004.4
(22) Anmeldetag: 17.04.2015
(51) Int. Cl.: A61K 9/06, A61K 8/02, A61K 8/81, A61K 31/785, A61P 31/02, A61P 31/04, A61Q 11/00

(54) **ORALES ABGABESYSTEM**
ORAL DELIVERY SYSTEM
SYSTÈME D'ADMINISTRATION PAR VOIE ORALE

(30) Priorität: 22.04.2014 DE 202014101882 U
(43) Veröffentlichungstag der Anmeldung: 01.03.2017
(73) Patentinhaber: Arnold, Christian, 85560 Ebersberg (DE); Armani, Armin, 82031 Grünwald (DE)
(72) Erfinder: Arnold, Christian, 85560 Ebersberg (DE); Armani, Armin, 82031 Grünwald (DE)
(74) Vertreter: advotec.
(86) Internationale Anmeldenummer: PCT/EP2015/058439
(87) Internationale Veröffentlichungsnummer: WO 2015/162070

(56) Entgegenhaltungen:
- DE-A1-102012 019 194
- US-A1- 2009 214 606
- US-A1- 2012 107 258

## Beschreibung

Die vorliegende Erfindung betrifft ein orales Abgabesystem für die Behandlung und/oder Vorbeugung von periodontalen, periimplantären sowie anderen bakteriellen und viralen Krankheiten oder Pilzerkrankungen im Mund- und Rachenraum sowie Halitosis (nachfolgend: erregerbedingte, pathologische Veränderungen).

Erregerbedingte, pathologische Veränderungen treten sehr häufig auf und sind der Hauptgrund für Zahnverlust/Implantatverlust und Halitosis bei Menschen in einem Alter von über 35 Jahren. Eine erregerbedingte, pathologische Veränderung kann z.B. als eine Infektion oder Entzündung der Zahnfleischtasche verstanden werden, welche, in mehreren Schritten, den Verlust des Knochens, welcher den Zahn/Implantat hält, bewirken kann. Es bestehen unterschiedliche Ausprägungen der Schwere der Krankheit. Leichtere Fälle betreffen die klinisch bezeichnete Gingivitis, während schwerwiegendere Fälle klinisch als Periodontitis/Periimplantitis bezeichnet werden.

Eine Gingivitis ist eine Entzündung der Gingiva (oder des Zahnfleisches), welche oft durch eine schlechte orale Hygiene und/oder den hormonalen Zustand des Patienten ausgelöst wird. Es wird angenommen, dass sich die unbehandelte Gingivitis in eine Periodontitis/Periimplantitis entwickelt. Die Periodontitis ist eine bakterielle Krankheit, welche das Zahnfleischgewebe, die Zähne, Implantate und den Knochen, welcher die Zähne/Implantate umgibt, angreift.

Die Mundhöhle ist eine im Wesentlichen aerobe Umgebung, welche durch den Speichel durchflossen ist. Im Gegensatz hierzu ist die periodontale/periimplantäre Mikroumgebung eher anaerob und wird durch ein Plasmafiltrat durchflossen, welches als "gingivale Sulkusflüssigkeit" bezeichnet wird. Das Wachstum von Mikroorganismen innerhalb dieser Mikroumgebung wird als verantwortlich für das Auftreten einer erregerbedingten, pathologischen Veränderung angesehen. Daher ist die Behandlung dieser Veränderung auf die Überwachung und Beeinflussung dieses Wachstums gerichtet.

Versuche zur Behandlung von erregerbedingten, pathologischen Veränderungen durch Wirkstoffe, z. B. antibakterielle Wirkstoffe, welche in die Mundhöhle gegeben werden, stellten sich in der Regel als unwirksam heraus, da die periodontale/periimplantäre Tasche im Wesentlichen nicht zugänglich ist. Andererseits führt die systemische Verabreichung von Antibiotika nur zu einem geringen Erfolg in der Behandlung von periodontalen Krankheiten.

Antibakterielle Wirkstoffe wie Chlorhexidin und quarternäre Ammoniumsalze in Form von Mundspülungen haben sich zwar als einigermaßen wirkungsvoll bei der Prophylaxe/Behandlung von erregerbedingten, pathologischen Veränderungen erwiesen. Diese Wirkstoffe haben jedoch verschiedene Nachteile. So treten häufig Nebenwirkungen, wie Verfärbung der Zähne, Zunge, Schleimhäute oder von Zahnersatz auf. Ferner weisen diese Wirkstoffe häufig üblen Geschmack auf und beeinträchtigen die Geschmacksempfindung des Patienten. Ferner kommt es bei diesen Wirkstoffen häufig zu Störungen der Wundheilung. Zudem tritt häufig auch Resorption der Wirkstoffe über die Schleimhäute oder über den Magen-Darm-Trakt auf, was zu systemischen Wirkungen führen kann. Ferner entstehen bei den genannten Wirkstoffen häufig toxische Abbauprodukte. Ein weiterer Nachteil besteht darin, dass die genannten Wirkstoffe meist in sehr hohen Konzentrationen eingesetzt werden müssen, um eine entsprechende Wirkung zu erzielen. Ferner wurde allergogenes Potenzial beobachtet. Außerdem wirken die genannten Wirkstoffe häufig irritativ und sind nicht besonders lange haltbar. Ferner wurde beobachtet, dass bei diesen Wirkstoffen die Wirkung durch Blut bzw. Eiweiß beeinflusst wird.

Es wurden pharmazeutische Zusammensetzungen, welche eine Freisetzung von Wirkstoffen aufweisen und welche in der Lage sind, in die periodontale Kavität eingesetzt zu werden und welche langsam einen antimikrobiellen Wirkstoff freisetzen, entwickelt. Zum Beispiel offenbaren US 4,764,377 und US 4,892,736 das Einbringen von Tetracyclin in nicht abbaubare polymere Fasern, welche um die Zähne herum gewickelt werden können und das Antibiotikum in der periodontalen Kavität für mehrere Tage freisetzen. Die Fasern müssen jedoch mit einem Klebstoff an ihrem Platz fixiert werden und am Ende der Behandlungsmethode wieder entfernt werden.

US 4,569,837 offenbart die Verwendung von wasserlöslichen polymeren Substanzen (z. B. Methylcellulose, Gelatine etc.) als eine polymere Matrix für ein periodontales Implantat.

US 5,002,769 offenbart ein biologisch abbaubares System zur oralen Verabreichung mit verzögerter Freisetzung für die Behandlung der periodontalen Krankheiten. Der Wirkstoff ist in eine Matrix von hydrolysierter Gelatine, welche mit Glutaraldehyd vernetzt ist, eingebettet.

DE102012019194 (A1) beschreibt ein medizinisches Präparat enthaltend a) ein Antiseptikum ausgewählt aus der Gruppe bestehend aus Polyhexanid, Octenidin und Mischungen davon und b) einen Träger.

US2009214606 (A1) beschreibt antimikrobielle, poröse Teilchen, die Wirkstoffe enthalten. Die porösen Teilchen können in eine Vielzahl von Produkten, z.B. Kaugummis, eingebracht werden. Zudem kann eine sehr große Anzahl an Wirkstoffen in den Teilchen enthalten sein, z.B. Polyhexanid.

US2012107258 (A1) beschreibt Formulierungen, die ein antimikrobielles Mittel enthalten zur Bekämpfung von Körpergeruch. Diese Formulierungen können in der Form eines Kaugummis sein. Polyhexanid ist in einer Liste von verwendbaren Konservierungsmitteln für dermatologische Zusammensetzungen erwähnt.

Die oben beschriebenen Zusammensetzungen haben unterschiedliche Wirksamkeiten beim Reduzieren der bakteriellen Menge der periodontalen Tasche und im Reduzieren der Taschentiefe. Darüber hinaus weisen diese Zusammensetzungen häufig die o.g. Nachteile auf

Der vorliegenden Erfindung liegt die Aufgabe zugrunde, ein wirksames Abgabesystem für die Behandlung und/oder Vorbeugung von erregerbedingten, pathologischen Veränderungen zur Verfügung zu stellen, welches einfach in der Anwendung und gleichzeitig effektiv ist.

Die Aufgabe wird gelöst durch ein orales Abgabesystem der eingangs genannten Art, welches Polyhexanid umfasst.

Es hat sich herausgestellt, dass Polyhexanid besonders effektiv beim Einsatz oraler Abgabesysteme bei der Behandlung und/oder Vorbeugung von periodontalen Krankheiten ist. Polyhexanid lässt sich insbesondere bei solchen Abgabesystemen einsetzen, bei welchen eine langsame Abgabe im Bereich der Zahnfleischtaschen erzielt werden soll, sodass eine verlängerte Einwirkzeit erreicht werden kann.

Erfindungsgemäß liegt das orale Abgabesystem in einer festen Darreichungsform in Form eines Kaugummis vor. Eine feste Darreichungsform hat insbesondere Vorteile bei der Verwendung des Systems.

Kaugummis haben u.a. den Vorteil, dass sie dem bei Polyhexanid beobachteten verzögerten Wirkeintritt entgegenwirken, da sie vom Patienten ohne Probleme über einen längeren Zeitraum benutzt werden können. Dies ist beispielsweise bei Mundspülungen problematisch.

Kaugummis sind im Allgemeinen aus folgenden Rohstoffgruppen zusammengesetzt: Gum base oder Kaumasse, Weichmacher, Füllstoffe, Gleitmittel, Fette, Emulgatoren, Aromen, Farbstoffe, Antioxidantien und Genuss-Säuren zum Aromatisieren.

Als Kaumasse können entweder Naturstoffe, wie Chicle, Gutta-percha, Latex, Benzoeharze oder Gummi arabicum, oder konsistenzgebende, synthetische Thermoplaste, wie Polyvinylacetat in Mengen von bis zu 65% der Kaumasse, Poly-Butadien-Styrol, Polyisobutylen, Isopren, Polyvinylether und Polyethylen, verwendet werden.

Typische Weichmacher sind Emulgatoren, Harze, Wachse oder Glucitol. Typische Füllstoffe sind Magnesiumstearat, Kreide, Calciumcarbonat, Silicat oder Cellulosen. Die Füllstoffe bzw. technischen Hilfsstoffe erhalten die Rieselfähigkeit bzw. verhindern das Verkleben der Partikel bei niedrigem Druck. Als Gleitmittel, Fette oder Emulgatoren kommen typischerweise Mineralöle, mikrokristalline Wachse oder pflanzliche Öle zum Einsatz. Diese Hilfsstoffe verhindern das Verkleben der Werkzeuge bzw. an den Werkzeugen.

Herkömmliche Kaugummis können aufgrund ihres hohen Zuckeranteils stark kariesfördernd sein, massieren aber auch das Zahnfleisch und die Speicheldrüsen bei Mundtrockenheit. Durch die zugesetzten Aromastoffe wirken Kaugummis auch erfrischend, belebend und/oder durststillend.

Um die oben genannte kariesfördernde Wirkung von Zucker in Kaugummis zu vermeiden, werden seit langem zuckerfreie Kaugummis auf dem Markt gebracht. Bei diesen wird der Zucker gegen Zuckeraustauschstoffe ersetzt. Dies sind vor allem Sorbit und Xylit.

Die erfindungsgemäßen Kaugummis dienen der unterstützenden Zahn- und Mundhygiene sowie der Behandlung/Vorbeugung von erregerbedingten, pathologischen Veränderungen. Sie sind insbesondere für unterwegs geeignet, wenn keine Möglichkeit zum Zähneputzen besteht. Die erfindungsgemäßen Kaugummis sind gewöhnlich zuckerfrei und enthalten, vergleichbar mit Zahnpasta, Spuren von Mineralien für die Regeneration der Zähne. Der erfindungsgemäße Kaugummi kann ebenfalls mindestens ein Schleifmittel oder mindestens einen Abrasivstoff, insbesondere Calziumcarbonat, Calziumphosphate, Metaphosphate, Kieselsäure, Aluminiumoxide, Silicate und/oder Talkum umfassen.

Des Weiteren kann der erfindungsgemäße Kaugummi mindestens einen Suspensionsstoff oder ein Feuchthaltemittel, insbesondere Wasser, Glycerin, Propylenglykol und/oder Sorbitsirup, sowie mindestens ein Verdickungs-, Stabilisierungs- und/oder Bindemittel, insbesondere Gele, Stärken, Alginate, Öle und/oder Cellulose Gum enthalten.

Zur Verbesserung der Geschmackseigenschaften können dem Kaugummi mindestens ein aromatischer Stoff, mindestens ein Süssungsmittel und/oder mindestens ein Zuckeraustauschstoff, insbesondere Menthol, Pfefferminzöl, Natriumsaccharin, Aspartame, Acesulfam, Sorbit, Malit, Xylit und/oder Fructose zugegeben werden.

Zudem kann der erfindungsgemäße Kaugummi weitere chemische Additive, Farbstoffe und/oder pH-Regulatoren, insbesondere Fluoride, Adstringentien, Entzündungshemmer, Desensiblisierungsmittel, Vitamine, Panthenol, Weißpigmente und/oder Natriumhydroxid enthalten.

Durch das erfindungsgemäße orale Abgabesystem in Form eines Kaugummis kann das Polyhexanid besonders effektiv an die Wirkorte, insbesondere in die Zahnfleischtaschen gelangen. Durch den Kauvorgang wird der Kaugummi in die Zahnfleischtasche gepresst, wo dann das Polyhexanid abgegeben wird.

Des Weiteren ist ein Abgabesystem in Form eines Chips oder Filmes offenbart, umfassend ein biologisch abbaubares oder bioerodierbares pharmazeutisch annehmbares Polymer.

Der Chip bzw. Film ist zum Implantieren in eine periodontale Tasche geeignet und in der Lage, erregerbedingte, pathologische Veränderungen zu behandeln, bei welchen eine verzögerte Abgabe von Polyhexanid gewünscht ist. Die Tasche kann eine natürliche Tasche sein, kann durch einen Krankheitszustand bedingt sein oder kann absichtlich geöffnet sein als Teil der Behandlung. Nach ihrer Implantation wird der Chip bzw. Film weich, quillt auf und verändert sich in eine weiche Paste, welche in der Tasche haftet.

Vorzugsweise kann der Chip bzw. Film mindestens ein Vernetzungsmittel umfassen, welches in einer Menge vorhanden ist, die ausreicht, um das Polymer wasserunlöslich zu machen, während die Freisetzung vom Polyhexanid aus dem Abgabesystem erlaubt wird.

Der Film bzw. Chip enthält vorzugsweise ein oberflächenaktives Mittel, welches vorzugsweise ausgewählt ist aus anionischen, kationischen und nicht-ionischen oberflächenaktiven Mitteln. Die nichtionischen oberflächenaktiven Mittel können ausgewählt sein aus Polyoxyethylen-Sorbitanfettsäureestern (Polysorbat) und Sorbitan-Fettsäureestem.

Der Chip bzw. Film ist vorzugsweise angepasst zur Verabreichung in eine periodontale Tasche mit in-vivo Freisetzungseigenschaften, welche darauf abzielen, die Tiefe einer periodontale Tasche eines Patienten zu reduzieren.

Mit Vorteil liegt der Chip bzw. Film in solch einer Form vor, dass er in der periodontale Tasche biologisch abgebaut wird, wobei er dadurch weich wird und an der periodontalen Tasche haftet und worin er, wenn er einmal in einer periodontalen Tasche eingesetzt wurde, graduell das Polyhexanid über einen Zeitraum von mindestens ca. 24 Stunden freisetzt, währenddessen sich der Chip bzw. Film in ein weiches Material umwandelt. Der Chip dient also als Abgabemedium von Polyhexanid als antimikrobieller Wirkstoff zur Applikation in den Sulkus oder Zahnfleischtasche. Die Größe eines Chips/Films liegt in der Regel bei 3x3 - 10x10 mm. In der Regel ist die Basis des Chips/Films eine mit Glutaraldehyd vernetzte Gelatine. Es hat sicher herausgestellt, dass vernetzte Gelatine besonders geeignet ist zur verzögerten Freisetzung von Polyhexanid.

Mit Vorteil ist das Polymer ausgewählt aus wasserlöslichem Protein, Zellulose oder Zellulose-Derivat, Stärke oder Stärke-Derivat, Glycerylmonostearat, Carbomer, PVP (Polyvinylpyrrolidon), Gummi, Akaziengummi, Guargummi, Polyvinylalkohol, Polyhydroxyethylmethacrylat, Polyhydroxymethylmethacrylatacrylsäure, Polyacrylamid, Polyethylenglykolen, Polyessigsäure, Polyglykolsäure, Copolymere von Polyessigsäure und Polyglykolsäure, Polyanhydride und Polyorthoestern. Das wasserlösliche Protein ist vorzugweise ausgewählt aus der Gruppe, bestehend aus Gelatine, Collagen, Albumin, einem Enzym und Fibrinogen.

Zudem ist ein Abgabesystem in der Form eines Gels oder Salbe offenbart. In dieser Form wird es in der Regel in die Zahnfleischtaschen verbracht, wo es Polyhexanid an die Umgebung abgibt.

Das Gel bzw. Salbe dient zur intraoralen Anwendung, insbesondere zur Applikation in den Sulkus oder in eine Zahnfleischtasche. Das Polyhexanid wirkt hier wiederum als antimikrobieller Wirkstoff. Das Gel/Salbe kann auf herkömmlicher Ethanol-/Glycerol-/Macrogol-Verbindung basieren.

Das Gel bzw. die Salbe kann sowohl als Fertigpräparat als auch als Mischsystem vorliegen. Beim Vorliegen eines Mischsystems werden bestimmte Komponenten erst kurz vor der Anwendung gemischt und an die erkrankte Stelle gebracht. Der Vorteil hierbei besteht u.a. darin, dass ein Mischsystem nach dem Mischen gut verarbeitbar ist. So kann bei einer Ausführungsform nach dem Mischen zunächst eine viskose Masse vorliegen, die erst nach dem Verbringen in den Mund aushärtet. Dies erleichtert die Anwendung erheblich. Das Mischsystem kann beispielsweise in Form einer Mischkapsel vorliegen.

Das erfindungsgemäße orale Abgabesystem hat gegenüber den bekannten antibakteriellen Wirkstoffen, wie beispielsweise Chlorhexidin gravierende Vorteile. So ist das im erfindungsgemäßen Abgabesystem eingesetzte Polyhexanid nicht zytotoxisch. Zudem sind keine Nebenwirkungen, wie Verfärbung der Zähne, Zunge, Schleimhäute oder von Zahnersatz zu beobachten. Das eingesetzte Polyhexanid ist geschmacksneutral und verursacht keine Geschmacksbeeinträchtigungen. Ferner wird die Wundheilung nicht gestört und die Fibrinbildung wird reduziert. Ferner ist keine Resorption über Schleimhäute oder über den Magen-Darm-Trakt bekannt. Ferner bilden sich bei der Anwendung keine toxischen Abbauprodukte. Ein weiterer erheblicher Vorteil besteht darin, dass die Wirkkonzentration von Polyhexanid um ein Vielfaches niedriger als beispielsweise von Chlorhexidin ist. Ferner birgt Polyhexanid kein allergogenes Potenzial, ist nicht irritativ. Polyhexanid ist - soweit bekannt - nicht sensibilisierend. Die Haltbarkeit von Polyhexanid ist ebenfalls länger als bei den herkömmlichen Wirkstoffen. Zudem wurde keine Resistenzbildung beobachtet. Polyhexanid hat ein breites Wirkspektrum und zeigt einen sehr geringen Eiweiß- und Blutfehler (Wirkung wird durch Eiweiß oder Blut kaum beeinträchtigt).

## Patentansprüche

1. Orales Abgabesystem für die Behandlung und/oder Vorbeugung von erregerbedingten, pathologischen Veränderungen im Mund- und Rachenraum, umfassend Polyhexanid, wobei das System in Form eines Kaugummis vorliegt.

2. Orales Abgabesystem nach Anspruch 1, **gekennzeichnet durch** mindestens einen Suspensionsstoff oder ein Feuchthaltemittel, insbesondere Wasser, Glycerin, Propylenglykol und/oder Sorbitsirup.

3. Orales Abgabesystem nach einem der Ansprüche 1 oder 2, **gekennzeichnet durch** mindestens ein Verdickungs-, Stabilisierungs- und/oder Bindemittel, insbesondere Gele, Stärken, Alginate, Öle und/oder Cellulose Gum.

4. Orales Abgabesystem nach einem der Ansprüche 1 bis 3, **gekennzeichnet durch** mindestens einen aromatischen Stoff, Süssungsmittel und/oder Zuckeraustauschstoffe, insbesondere Menthol, Pfefferminzöl, Natriumsaccharin, Aspartam, Acesulfam, Sorbit, Malit, Xylit und/oder Fructose.

5. Orales Abgabesystem nach einem der Ansprüche 1 bis 4, **gekennzeichnet durch** weitere chemische Additive, Farbstoffe und/oder pH- Regulatoren, insbesondere Fluoride, Adstringentien, Desensibilisierungsmittel, Vitamine, Panthenol, Weißpigmente und/oder Natriumhydroxid.

6. Orales Abgabesystem nach einem der Ansprüche 1 bis 5, zur Verwendung in einem Verfahren der Behandlung und/oder Vorbeugung von erregerbedingten, pathologischen Veränderungen im Mund- und Rachenraum.

## Claims

1. An oral delivery system for the treatment and/or prevention of infectious pathological changes in the area of the mouth and throat, comprising polyhexanide, the system being in the form of a chewing gum.

2. The oral delivery system according to claim 1,
**characterized by**
at least one suspending agent or a humectant, in particular water, glycerin, propylene glycol and/or sorbitol syrup.

3. The oral delivery system according to any one of claims 1 or 2,
**characterized by**
at least one thickening agent, stabilizer and/or binder, in particular gels, starches, alginates, oils and/or cellulose gum.

4. The oral delivery system according to any one of claims 1 to 3,
**characterized by**
at least one aromatic substance, sweetener and/or sugar substitutes, in particular menthol, peppermint oil, sodium saccharin, aspartame, acesulfame, sorbitol, maltitol, xylitol and/or fructose.

5. The oral delivery system according to any one of claims 1 to 4,
**characterized by**
further chemical additives, dyes and/or pH regulators, in particular fluorides, astringents, desensitizers, vitamins, panthenol, white pigments and/or sodium hydroxide.

6. The oral delivery system according to any one of claims 1 to 5 for use in a method of treating and/or preventing infectious pathological changes in the area of the mouth and throat.

## Revendications

1. Système d'administration orale pour le traitement et/ou la prévention des altérations pathologiques induites par des agents pathogènes dans la cavité bucco-pharyngée, comprenant du polyhexanide, le système étant sous la forme d'une gomme à mâcher.

2. Système d'administration orale selon la revendication 1,
**caractérisé par**
au moins un agent de suspension ou un agent humectant, notamment de l'eau, de la glycérine, du propylène glycol et/ou du sirop de sorbitol.

3. Système d'administration orale selon l'une quelconque des revendications 1 ou 2,
**caractérisé par**
au moins un épaississant, un agent stabilisant et/ou un liant, notamment des gels, des amidons, des alginates, des huiles et/ou de la gomme cellulose.

4. Système d'administration orale selon l'une quelconque des revendications 1 à 3,
**caractérisé par**
au moins une substance aromatique, des édulcorants et/ou des substituts de sucre, notamment du menthol, de l'huile de menthe, de la saccharine de sodium, de l'aspartame, de l'acésulfame, du sorbitol, du maltitol, du xylitol et/ou du fructose.

5. Système d'administration orale selon l'une quelconque des revendications 1 à 4,
**caractérisé par**
d'autres additifs chimiques, des colorants et/ou des régulateurs de pH, notamment des fluorures, des astringents, des agents désensibilisants, des vitamines, du panthénol, des pigments blancs et/ou de l'hydroxyde de sodium.

6. Système d'administration orale selon l'une quelconque des revendications 1 à 5 pour l'usage dans un procédé de traitement et/ou de prévention des altérations pathologiques induites par des agents pathogènes dans la cavité bucco-pharyngée.
